# NOUVEAU FASCICULE DE BREVET EUROPEEN

(11) **EP 1 084 702 B2**
(45) Date de publication et mention de la décision concernant l'opposition: **20.07.2005**
(45) Mention de la délivrance du brevet: 12.06.2002
(21) Numéro de dépôt: 00402251.3
(22) Date de dépôt: 09.08.2000
(51) Int. Cl.: A61K 7/48, A61K 7/06

(54) **Composition cosmétique, notamment pour le nettoyage de la peau**
Kosmetische Zusammensetzung insbesondere zur Reinigung der Haut
Cosmetic composition particularly for skin cleansing

(30) Priorité: 15.09.1999 FR 9911521
(43) Date de publication de la demande: 21.03.2001
(73) Titulaire: L'OREAL, 75008 Paris (FR)
(72) Inventeur: Guillou, Véronique, 92160 Antony (FR); Sebillotte-Arnaud, Laurence, 94240 L'Hay Les Roses (FR)
(74) Mandataire: Rasson, Catherine

(56) Documents cités:
- EP-A- 0 668 072
- WO-A-98/26758
- JP-A- 6 465 197
- US-A- 2 972 582
- US-A- 5 071 960
- US-A- 5 635 469
- ALLISON W C ET AL: "AMMONIUM COCOYL ISETHIONATE: A NEW, MILD, HIGH-FOAMING SURFACTANT FOR PERSONAL CARE A NEW AND USEFUL INGREDIENT FOR THE FORMULATOR OF CLEAR, HIGH-FOAMING SYSTEMS THAT DELIVERS CONSUMER-PREFERRED PROPERTIES OFTHE SODIUM COCOYL ISETHIONATE SURFACTANT" HAPPI HOUSEHOLD AND PERSONAL PRODUCTS INDUSTRY,US,DORLAND PUBLISHING, DENVILLE, NJ, vol. 32, no. 5, 1 mai 1995 (1995-05-01), page 92,94,96,98, XP000498911 ISSN: 0090-8878
- ARIOTTO A ET AL: "SKIN AND HAIR BENEFIT FROM WHEAT PROTEIN" MANUFACTURING CHEMIST,GB,MORGAN-GRAMPIAN LTD. LONDON, vol. 67, no. 2, 1 février 1996 (1996-02-01), pages 23-25, XP000682299 ISSN: 0262-4230
- Manufacturing Chemist, February 1996, pages 23-25

## Description

L'invention a pour objet des compositions aqueuses transparentes à base d'acyliséthionate et d'hydrolysat de protéine, et l'utilisation des dites compositions notamment dans le domaine cosmétique pour le nettoyage de la peau, des cheveux, des lèvres et/ou des yeux, ainsi qu'à l'utilisation d'un hydrolysat de protéine pour la stabilisation d'un acyliséthionate dans une composition aqueuse.

Le nettoyage de la peau est très important pour le soin du visage. Il doit être le plus performant possible car les résidus gras tels que l'excès de sébum, les restes des produits cosmétiques utilisés quotidiennement et les produits de maquillage, notamment les produits "waterproof", résistants à l'eau, s'accumulent dans les replis cutanés et à la surface de la peau et peuvent obstruer les pores de la peau et entraîner l'apparition de boutons. Une mauvaise qualité de nettoyage est souvent responsable parmi d'autres facteurs de cause, d'un teint brouillé.

Parmi les produits de nettoyage de la peau, on utilise couramment des nettoyants moussants qui contiennent généralement des tensioactifs anioniques. On cherche notamment à faire des nettoyants moussants transparents car, tout comme l'eau, la transparence est le symbole de pureté, et donc de propreté. Comme tensioactifs anioniques, on peut utiliser en particulier des acyliséthionates tels que le cocoyliséthionate, qui sont particulièrement intéressants car ils permettent d'obtenir une mousse dense, fine et volumineuse. Cependant, ces acyliséthionates présentent l'inconvénient d'être instables en solution aqueuse Ils recristallisent dans le temps, et ce d'autant plus vite que la température est basse (c'est-à-dire inférieure ou égale à l'ambiante). Du fait de cette instabilité, les gels contenant des iséthionates ne peuvent pas être transparents. Par conséquent, il est impossible de formuler des nettoyants moussants transparents stables contenant un acyliséthionate.

Selon le document WO-98/26758, il est remédié à cet inconvénient en ajoutant un tensioactif amphotère tels qu'un dérivé d'imidazoline ou une bêtaïne. Toutefois, pour atteindre une parfaite solubilité de l'iséthionate, un taux élevé de tensioactif amphotère est nécessaire et ainsi, le rapport en poids amphotère/iséthionate doit être supérieur à 1. L'introduction d'un amphotère à un taux tel que proposé par ce document fait perdre les performances intéressantes du cocoyliséthionate, à savoir la densité et l'onctuosité de la mousse.

Il subsiste donc le besoin d'une composition aqueuse contenant un acyliséthionate, stable dans le temps, transparente, et n'ayant pas les inconvénients de l'art antérieur.

La demanderesse a trouvé de manière surprenante que la recristallisation des acyliséthionates pouvait être évitée grâce à l'utilisation d'hydrolysats de protéine à groupement hydrophobe conforme aux revendications. En outre, ces composés permettent de garder à la mousse les qualités obtenues avec le cocoyliséthionate, c'est-à-dire finesse, densité et onctuosité de la mousse.

Certes, le document US-A-2,972,582 divulgue l'association d'acyliséthionate et de polypeptide acylé. Toutefois, ce document n'enseigne pas que les polypeptides acylés permettent de solubiliser l'acyliséthionate, et, par ailleurs, ce document décrit des compositions opaques dans lesquelles le polypeptide est en quantité insuffisante pour solubiliser l'acyliséthionate, et des compositions contenant de l'huile (lanoline) qui ont l'inconvénient de laisser sur la peau un dépôt huileux, ce que l'on veut éviter en utilisant une composition aqueuse exempte d'huile.

L'invention a ainsi pour objet une composition aqueuse transparente, conforme à la revendication 1.

Par « composition aqueuse », on entend une composition contenant de l'eau et exempte d'huile.

Le mot « transparent » signifie qu'au travers d'une bouteille transparente contenant la composition, on peut distinguer les caractères imprimés sur une page de journal placée derrière cette bouteille.

On entend ici par « milieu physiologiquement acceptable », un milieu compatible avec la peau, les lèvres, le cuir chevelu, les cils, les yeux et/ou les cheveux.

Par ailleurs, on entend par « hydrolysat de protéine à groupement hydrophobe » un dérivé d'hydrolyse d'une protéine comportant un groupement hydrophobe, ledit groupement hydrophobe pouvant être naturellement présent dans la protéine ou être ajouté par réaction de la protéine et/ou de l'hydrolysat de protéine avec un composé hydrophobe.

La présence de l'hydrolysat de protéine à groupement hydrophobe permet d'éviter la recristallisation de l'acyliséthionate. En outre, le mélange d'acyliséthionate et d'hydrolysat de protéine donne une qualité de mousse beaucoup plus intéressante que le mélange d'acyliséthionate et d'amphotère, décrit dans l'art antérieur, c'est-à-dire une mousse plus fine et plus dense. De plus, le produit obtenu se rince plus facilement et ne laisse pas de film comme le font les produits de l'art antérieur.

La quantité totale d'hydrolysat de protéine à groupement hydrophobe dans la composition de l'invention doit être suffisante pour solubiliser l'acyliséthionate. En pratique, elle est au moins égale et de préférence supérieure à la quantité en poids d'acyliséthionate. Elle va de préférence de 0,5 à 30 % en poids de matière active, et plus préférentiellement de 1 à 10 % en poids de matière active par rapport au poids total de la composition.

L'invention a encore pour objet l'utilisation d'une quantité efficace d'au moins un hydrolysat de protéine à groupement hydrophobe, l'hydrolysat de protéine étant un hydrolysat de protéine d'origine végétale ou un hydrolysat de protéine de soie pour la stabilisation d'un acyliséthionate dans une composition aqueuse.

L'acyliséthionate utilisable dans la composition de l'invention est un composé de formule (I) :

RCO₂(CH₂)ₙSO₃⁻ M⁺ (I)

dans laquelle R est un radical hydrocarboné comportant de 6 à 26 atomes de carbone, est un nombre entier de 2 à 4 et M est un métal alcalin ou alcalino-terreux tel que le sodium, le potassium, le lithium et le magnésium.

Selon un mode préféré de réalisation de l'invention, le radical R dans la formule (I) comporte de 8 à 22 atomes de carbone et est choisi parmi les radicaux lauroyl, palmitoyl, caproyl, capryloyl, myristoyl, stéaroyl, arachidoyl, oleyl, et les mélanges de tels radicaux tels que le radical cocoyl. M est de préférence le sodium, le potassium.

Selon un mode particulièrement préféré de réalisation de l'invention, l'acyliséthionate utilisé dans la composition de l'invention est le cocoyliséthionate de sodium ou de potassium.

L'acyliséthionate est présent dans la composition de l'invention en une quantité allant de 0,5 à 10% et de préférence de 1 à 10 % en poids par rapport au poids total de la composition.

Comme indiqué ci-dessus, l'hydrolysat de protéine à groupement hydrophobe est un dérivé d'hydrolyse d'une protéine comportant un groupement hydrophobe, ledit groupement hydrophobe pouvant être naturellement présent dans la protéine ou être ajouté par réaction de la protéine et/ou de l'hydrolysat de protéine avec un composé hydrophobe. Les protéines peuvent être d'origine végétale ou de soie, celles d'origine végétale étant préférées, et elles peuvent être quaternisées ou non quaternisées, ioniques ou non ioniques. Le groupement hydrophobe peut être notamment une chaîne grasse, par exemple une chaîne alkyle comportant de 8 à 22 atomes de carbone.

Comme hydrolysats de protéine à groupement hydrophobe, utilisables dans la composition selon l'invention; on peut plus particulièrement citer les hydrolysats de protéines d'origine végétale, en particulier ceux des protéines de blé, de soja, d'avoine ou les hydrolysats de protéine de soie; comportant une chaîne alkyle ayant de 8 à 22 atomes de carbone, et les dérivés ioniques de ces composés. La chaîne alkyle peut être notamment une chaîne lauryle et le dérivé ionique un sel de sodium, de potassium et/ou d'ammonium. On peut citer par exemple les hydrolysats de protéine de soie modifiée par l'acide laurique, et leurs sels de sodium, de potassium et/ou d'ammonium, tels que le produit commercialisé sous la dénomination KAWA SILK par la société Kawaken, les hydrolysats de protéine de blé modifiée par l'acide laurique et leurs sels de sodium, de potassium et/ou d'ammonium, tels que le produit commercialisé sous la dénomination AMINOFOAM W OR par la société Croda et celui commercialisé sous la dénomination PROTEOL LW 30 par la société Seppic, les hydrolysats de protéine d'avoine modifiée par l'acide laurique et leurs sels de sodium, de potassium et/ou d'ammonium, tels que le produit commercialisé sous la dénomination PROTEOL OAT par la société Seppic.

Le milieu aqueux cosmétiquement acceptable de la composition de l'invention peut être constitué uniquement par de l'eau ou par un mélange d'eau et d'un solvant cosmétiquement acceptable tel qu'un alcool inférieur en C₁-C₄, comme l'éthanol, l'isopropanol, le tertiobutanol, le n-butanol ; les polyols comme le propylène glycol ; les éthers de glycols.

L'eau est présente dans la composition de l'invention de préférence en une quantité allant de 40 à 95 % en poids par rapport au poids total de la composition. Avantageusement l'eau représente de 50 à 85 % en poids par rapport au poids total de la composition.

La quantité de solvant(s) peut aller par exemple de 1 à 30 % en poids, et de préférence de 2 à 20 % en poids par rapport au poids total de la composition.

Habituellement on entend par eau de l'eau pure. Toutefois, une partie de l'eau utilisée dans les compositions selon l'invention peut éventuellement être choisie parmi les eaux minérales ou thermales. En général, une eau minérale est propre à la consommation, ce qui n'est pas toujours le cas d'une eau thermale. Chacune de ces eaux contient, entre autre, des minéraux solubilisés et des oligo-éléments. Ces eaux sont connues pour être employées à des fins de traitement spécifique selon les oligo-éléments et les minéraux particuliers qu'elles contiennent, tels que l'hydratation et la désensibilisation de la peau ou le traitement de certaines dermatoses. Par eaux minérales ou thermales, on désignera non seulement les eaux minérales ou thermales naturelles, mais également des eaux minérales ou thermales naturelles enrichies en constituants minéraux et/ou en oligo-éléments supplémentaires, ainsi que des solutions aqueuses minérales et/ou oligo-élémentaires préparées à partir d'eau purifiée (déminéralisée ou distillée).

Une eau thermale ou minérale naturelle utilisée selon l'invention peut, par exemple être choisie parmi l'eau de Vittel, les eaux du bassin de Vichy, l'eau d'Uriage, l'eau de La Roche Posay, l'eau de La Bourboule, l'eau d'Enghien-les-Bains, l'eau de Saint Gervais-les-Bains, l'eau de Néris-les-Bains, l'eau d'Allevard-les-Bains, l'eau de Digne, l'eau de Maizières, l'eau de Neyrac-les-Bains, l'eau de Lons-le-Saunier, l'eau des Eaux Bonnes, l'eau de Rochefort, l'eau de Saint Christau, l'eau des Fumades et l'eau de Tercis-les-bains, l'eau d'Uriage-les-bains, l'eau d'Avene.

Les compositions selon l'invention présentent un pH final allant généralement de 3 à 10. De préférence, ce pH va de 5 à 8. L'ajustement du pH à la valeur désirée peut se faire classiquement par ajout d'une base (organique ou minérale) dans la composition, par exemple de la soude, de l'ammoniaque ou une (poly) amine primaire, secondaire ou tertiaire comme la monoéthanolamine, la diéthanolamine, la triéthanolamine, l'isopropanolamine ou la propanediamine-1,3, ou encore par ajout d'un acide organique ou minéral tel que par exemple l'acide citrique, l'acide lactique et l'acide chlorhydrique.

Selon un mode préféré de réalisation de l'invention, les compositions selon l'invention peuvent contenir un tensioactif moussant autre que l'acyliséthionate et l'hydrolysat de protéine à groupement hydrophobe. On peut se reporter au document « Encyclopedia of Chemical Technology, KIRK-OTHMER », volume 22, p. 333-432, 3ème édition, 1979, WILEY, où sont citées les principales classes de tensioactifs connues de l'homme du métier ainsi que leurs fonctions (en particulier le fait d'être moussant).

Les tensioactifs moussants peuvent être choisis notamment parmi les tensioactifs anioniques, amphotères et non-ioniques. Parmi ces classes de tensioactifs, on peut citer par exemple :
- comme tensioactifs amphotères : les alkylbétaïnes tels que la diméthylbétaïne, la coco-bétaïne ; les alkylsulfobétaïnes ; les alkylamidopropylbétaïnes tels que la coco-amidopropylbétaïne, les alkylsultaïnes tels que la cocoamidopropylhydroxysultaïne ; les alkylcarboxyglycinates de métal alcalin ou alcalino-terreux ; les imidazolines ; les amphoacétates tels que le di-sodium cocoamphodiacétate ;
- comme tensioactifs anioniques: les alkyl phosphates tels que le laurylphosphate de sodium ; les alkyl taurates tels que le méthylpalmitoyltaurate de sodium ; les sulfosuccinates tels que le sulfosuccinate de cocoyle, le sel disodique du sulfosuccinate d'alcool laurique oxyéthyléné ; les alkyl sulfates tels que le lauryl sulfate de triéthanolamine ; les sarcosinates tels que le lauroyl sarcosinate de sodium ; les alkyl éthersulfates tels que le lauroyl éther sulfate de sodium ; les alkyléther carboxylates tels que le décyl éther carboxylate de sodium oxyéthyléné ;
- comme tensioactifs non-ioniques : les composés de formule:

   R¹O(G)ₒ

   dans laquelle R¹ est une chaîne hydrocarbonée monovalent contenant de 1 à 30 atomes de carbone, G est un dérivé de saccharide contenant de 5 à 6 atomes de carbone et p est une valeur moyenne statistique allant de 1 à 6 ; les composés de formule (II) sont décrits notamment dans la demande WO 94/27562. Comme composés préférentiels, on peut citer par exemple les alkylpolyglycosides tels que le décylglucoside et le laurylglucoside commercialisés par exemple par la société Henkel sous les dénominations respectives Plantaren 2000, Plantaren 1200 et Plantacare 2000 UP.

On utilise de préférence comme autre tensioactif moussant, un alkylpolyglycoside et plus particulièrement le décylglucoside ou le laurylglucoside ou leurs mélanges. L'addition d'alkylpolyglycoside dans la composition de l'invention a l'avantage d'augmenter le volume de la mousse obtenue avec ladite composition, tout en donnant une composition très bien tolérée et qui se rince facilement.

Selon ce mode de réalisation, l'invention a pour objet une composition aqueuse transparente comprenant dans un milieu physiologiquement acceptable, (i) au moins un acyliséthionate tel que défini ci-dessus, (ii) au moins un hydrolysat de protéine à groupement hydrophobe en une quantité suffisante pour solubiliser l'acyliséthionate la quantité en poids de matière active d'hydrolysat de protéine à groupement hydrophobe étant au moins égale à la quantité en poids d'acyliséthionate, l'hydrolysat de protéine étant un hydrolysat de protéine d'origine végétale ou un hydrolysat de protéine de soie et (iii) un alkylpolyglucoside.

Les tensioactifs moussants autres que l'acyliséthionate et l'hydrolysat de protéine à groupement hydrophobe, peuvent être présents en une quantité allant de 0,5 à 15 % en poids et de préférence de 1 à 10 % en poids par rapport au poids total de la composition.

Les compositions de l'invention peuvent, en outre, contenir des adjuvants hydrosolubles ou liposolubles habituels dans le domaine cosmétique tels que les conservateurs, les antioxydants, les parfums, les matières colorantes, les nacres, les actifs hydrophiles ou lipophiles, les agents régulateurs de viscosité et épaississants, ou d'autres agents ayant pour effet d'améliorer les propriétés cosmétiques de cheveux ou de la peau, tels que les polymères anioniques, non ioniques, cationiques ou amphotères. Ces adjuvants ainsi que leurs concentrations doivent être tels qu'ils ne modifient pas la propriété recherchée pour la composition.

Comme actifs, on peut citer par exemple les agents hydratants et notamment les polyols tels que le propylène glycol, le 1,3-butylène glycol, la glycérine, les polyglycérines, les sucres et le sorbitol ; les céramides et pseudocéramides ; les hydroxyacides ; les vitamines ; les agents antipelliculaires ou antiséborrhéiques ; les filtres solaires ; les agents anti-radicaux libres.

Comme agents régulateurs de viscosité, on peur citer notamment les alcools et esters gras alcoxylés tels que les produits Ceteareth-60 myristyl glycol, PEG-120 methyl glucose dioleate, PEG-150 pentaerythrityl tetrastearate (nom CTFA).

La composition peut constituer notamment une composition cosmétique, utilisable plus particulièrement pour le nettoyage et/ou de démaquillage de la peau, des cheveux, des lèvres et/ou des yeux. Elle peut constituer plus spécialement un gel nettoyant moussant transparent.

La présente invention a encore pour objet un procédé cosmétique de démaquillage et/ou de nettoyage de la peau, des cheveux, des lèvres et/ou des yeux, caractérisé par le fait que l'on applique sur la peau, les cheveux, les lèvres et/ou les yeux, une composition telle que définie ci-dessus.

L'invention a aussi pour objet l'utilisation cosmétique de la composition telle que définie ci-dessus pour le démaquillage et/ou le nettoyage de la peau, des cheveux, des lèvres et/ou des yeux.

L'exemple ci-après de compositions selon l'invention est donné à titre d'illustration et sans caractère limitatif. Les composés y sont donnés en nom CTFA et les quantités y sont données en % en poids de matière active, sauf mention contraire.

### Exemple : Gel moussant

- Sodium cocoyl isethionate (Jordapon Cl P® de la société Jordan) 3,25 %
- Decylglucoside (Plantacare 2000 UP® de la société Henkel) 3,25 %
- Potassium lauroyl wheat aminoacids (Aminofoam W OR® de la société Croda) 3,25 %
- PEG-120 methyl glucose dioleate (Glucamate DOE-120 vegetal® de la société Amerchol)4 %
- Sorbitol 3,5 %
- Glycérine 3,5 %
- Conservateurs 0,5 %
- Eau qsp 100 %

On obtient un gel fluide transparent, de viscosité de l'ordre de 500 mPa.s mesurée au Rhéomat RM 180 mobile 3 à 25°C, dont le pouvoir moussant est satisfaisant.

## Revendications

1. Composition aqueuse transparente comprenant dans un milieu physiologiquement acceptable, (i) au moins un acyliséthionate de formule (I) :
RCO₂(CH₂)ₙSO₃⁻ M⁺
dans laquelle R est un radical hydrocarboné comportant de 6 à 26 atomes de carbone, n est un nombre entier de 2 à 4 et M est un métal alcalin ou alcalino-terreux, l'acyliséthionate étant présent en une quantité allant de 0,5 à 10 % en poids par rapport au poids total de la composition,
et (ii) au moins un hydrolysat de protéine à groupement hydrophobe en une quantité suffisante pour solubiliser l'acyliséthionate, la quantité en poids de matière active d'hydrolysat de protéine à groupement hydrophobe étant au moins égale à la quantité en poids d'acyliséthionate, et l'hydrolysat de protéine étant un hydrolysat de protéine d'origine végétale ou un hydrolysat de protéine de soie.

2. Composition selon la revendication précédente, **caractérisée en ce que** dans la formule (I), R est le radical cocoyl et M est le sodium ou le potassium.

3. Composition selon l'une quelconque des revendications précédentes, **caractérisée en ce que** le groupement hydrophobe de l'hydrolysat de protéine est une chaîne alkyle ayant de 8 à 22 atomes de carbone.

4. Composition selon l'une quelconque des revendications précédentes, **caractérisée en ce que** le groupement hydrophobe est une chaîne lauryle.

5. Composition selon l'une quelconque des revendications précédentes, **caractérisée en ce que** l'hydrolysat de protéine à groupement hydrophobe est choisi parmi les hydrolysats de soja, de blé et d'avoine.

6. Composition selon la revendication précédente, **caractérisée en ce que** l'hydrolysat de protéine à groupement hydrophobe est choisi parmi les les hydrolysats de protéine de blé modifié par l'acide laurique, les hydrolysats de protéine d'avoine modifiée par l'acide laurique, leurs sels et leurs mélanges.

7. Composition selon l'une quelconque des revendications précédentes, **caractérisée en ce que** la quantité d'hydrolysat de protéine à groupement hydrophobe va de 0,5 à 30 % en poids de matière active par rapport au poids total de la composition.

8. Composition selon l'une quelconque des revendications précédentes, **caractérisée en ce que** l'eau est présente en une quantité allant de 40 à 95 % en poids par rapport au poids total de la composition.

9. Composition selon l'une quelconque des revendications précédentes, **caractérisée en ce qu'**elle contient en outre un autre tensioactif moussant.

10. Composition selon la revendication précédente, **caractérisée en ce que** le tensioactif moussant est un tensioactif non-ionique.

11. Composition selon la revendication précédente, **caractérisée en ce que** le tensioactif non-ionique est choisi parmi les composés de formule :
R¹O(G)ₚ
dans laquelle R¹ est une chaîne hydrocarbonée monovalent contenant de 1 à 30 atomes de carbone, G est un dérivé de saccharide contenant de 5 à 6 atomes de carbone et p est une valeur moyenne statistique allant de 1 à 6.

12. Composition selon la revendication 10 ou 11, **caractérisée en ce que** le tensioactif non-ionique est choisi parmi le décylglucoside, le laurylglucoside et leurs mélanges.

13. Composition aqueuse transparente comprenant dans un milieu physiologiquement acceptable, (i) au moins un acyliséthionate de formule (I) :
RCO₂(CH₂)ₙSO₃⁻M⁺
dans laquelle R est un radical hydrocarboné comportant de 6 à 26 atomes de carbone, n est un nombre entier de 2 à 4 et M est un métal alcalin ou alcalino-terreux, l'acyliséthionate étant présent en une quantité allant de 0,5 à 10 % en poids par rapport au poids total de la composition, (ii) au moins un hydrolysat de protéine à groupement hydrophobe en une quantité suffisante pour solubiliser l'acyliséthionate, la quantité en poids de matière active d'hydrolysat de protéine à groupement hydrophobe étant au moins égale à la quantité en poids d'acyliséthionate, l'hydrolysat de protéine étant un hydrolysat de protéine d'origine végétale ou un hydrolysat de protéine de soie et (iii) un alkylpolyglucoside.

14. Composition selon l'une quelconque des revendications précédentes, **caractérisée en ce qu'**elle constitue une composition cosmétique.

15. Composition selon l'une quelconque des revendications précédentes, **caractérisée en ce qu'**elle constitue un gel transparent.

16. Procédé cosmétique de démaquillage et/ou de nettoyage de la peau, des cheveux, des lèvres et/ou des yeux, **caractérisé par le fait que** l'on applique sur la peau, les cheveux, les lèvres et/ou les yeux, une composition selon l'une quelconque des revendications précédentes.

17. Utilisation cosmétique de la composition selon l'une quelconque des revendications 1 à 15 pour le démaquillage et/ou le nettoyage de la peau, des cheveux, des lèvres et/ou des yeux.

18. Utilisation d'au moins un hydrolysat de protéine à groupement hydrophobe, l'hydrolysat de protéine étant un hydrolysat de protéine d'origine végétale ou un hydrolysat de protéine de soie, pour la stabilisation d'un acyliséthionate dans une composition aqueuse.

## Patentansprüche

1. Transparente wässrige Zusammensetzung, die in einem physiologisch akzeptablen Medium (i) mindestens ein Acylisethionat der Formel (I):
RCO₂(CH₂)ₙSO₃⁻M⁺,
wobei R eine Kohlenwasserstoffgruppe mit 6 bis 26 Kohlenstoffatomen, n eine ganze Zahl im Bereich von 2 bis 4 und M ein Alkali- oder Erdalkalimetall bedeutet und wobei das Isethionat in einer Menge vom 0,5 bis 10 Gew.-%, bezogen auf das Gesamtgewicht der Zusammensetzung, vorliegt,
und (ii) mindestens ein Proteinhydrolysat mit hydrophober Gruppe in einer zur Solubilisierung des Acylisethionats ausreichenden Menge enthält, wobei die als Wirkstoff ausgedrückte Gewichtsmenge des Proteinhydrolysats mit hydrophober Gruppe mindestens der Gewichtsmenge des Acylisethionats entspricht und wobei das Proteinhydrolysat ein Proteinhydrolysat pflanzlicher Herkunft oder ein Seidenproteinhydrolysat ist.

2. Zusammensetzung nach dem vorhergehenden Anspruch, **dadurch gekennzeichnet, dass** in der Formel (I) R Cocoyl und M Natrium oder Kalium bedeutet.

3. Zusammensetzung nach einem der vorhergehenden Ansprüche, **dadurch gekennzeichnet, dass** die hydrophobe Gruppe des Proteinhydrolysats eine Alkylgruppe mit 8 bis 22 Kohlenstoffatomen ist.

4. Zusammensetzung nach einem der vorhergehenden Ansprüche, **dadurch gekennzeichnet, dass** die hydrophobe Gruppe die Laurylgruppe ist.

5. Zusammensetzung nach einem der vorhergehenden Ansprüche, **dadurch gekennzeichnet, dass** das Proteinhydrolysat mit hydrophober Gruppe unter den Sojaprotein-, Weizenprotein- und Haferprotein-Hydrolysaten ausgewählt ist.

6. Zusammensetzung nach dem vorhergehenden Anspruch, **dadurch gekennzeichnet, dass** das Proteinhydrolysat mit hydrophober Gruppe unter den Hydrolysaten vom mit Laurinsäure modifiziertem Weizenprotein, Hydrolysaten vom mit Laurinsäure modifiziertem Haferprotein und ihren Salzen und Gemischen ausgewählt ist.

7. Zusammensetzung nach einem der vorhergehenden Ansprüche, **dadurch gekennzeichnet, dass** der Mengenanteil des Proteinhydrolysats mit hydrophober Gruppe im Bereich von 0,5 bis 30 Gew.-% Wirkstoff, bezogen auf das Gesamtgewicht der Zusammensetzung, liegt.

8. Zusammensetzung nach einem der vorhergehenden Ansprüche, **dadurch gekennzeichnet, dass** das Wasser in einer Menge von 40 bis 95 Gew.-%, bezogen auf das Gesamtgewicht der Zusammensetzung, vorliegt.

9. Zusammensetzung nach einem der vorhergehenden Ansprüche, **dadurch gekennzeichnet, dass** sie außerdem einen schäumenden grenzflächenaktiven Stoff enthält.

10. Zusammensetzung nach dem vorhergehenden Anspruch, **dadurch gekennzeichnet, dass** der schäumende grenzflächenaktive Stoff ein nichtionischer grenzflächenaktiver Stoff ist.

11. Zusammensetzung nach dem vorhergehenden Anspruch, **dadurch gekennzeichnet, dass** der nichtionische grenzflächenaktive Stoff unter den Verbindungen der folgenden Formel ausgewählt ist:
R¹O(G)ₚ,
wobei R¹ eine einwertige Kohlenwasserstoffgruppe mit 1 bis 30 Kohlenstoffatomen, G ein Saccharidderivat mit 5 bis 6 Kohlenstoffatomen und p einen statistischen Mittelwert im Bereich von 1 bis 6 bedeutet.

12. Zusammensetzung nach Anspruch 10 oder 11, **dadurch gekennzeichnet, dass** der nichtionische grenzflächenaktive Stoff unter Decylglucosid und Laurylglucosid und deren Gemischen ausgewählt ist.

13. Transparente wässrige Zusammensetzung, die in einem physiologisch akzeptablen Medium (i) mindestens ein Acylisethionat der Formel (I)
RCO₂(CH₂)ₙSO₃⁻M⁺,
wobei R eine Kohlenwasserstoffgruppe mit 6 bis 26 Kohlenstoffatomen, n eine ganze Zahl von 2 bis 4 und M ein Alkali- oder Erdalkalimetall bedeutet und wobei das Isethionat in einer Menge vom 0,5 bis 10 Gew.-%, bezogen auf das Gesamtgewicht der Zusammensetzung, vorliegt, (ii) mindestens ein Proteinhydrolysat mit hydrophober Gruppe in einer zur Solubilisierung des Acylisethionats ausreichenden Menge, wobei die als Wirkstoff ausgedrückte Gewichtsmenge des Proteinhydrolysats mit hydrophober Gruppe mindestens der Gewichtsmenge des Acylisethionats entspricht, wobei das Proteinhydrolysat ein Proteinhydrolysat pflanzlicher Herkunft oder ein Seidenproteinhydrolysat ist, und (iii) ein Alkylpolyglycosid enthält.

14. Zusammensetzung nach dem vorhergehenden Anspruch, **dadurch gekennzeichnet, dass** sie eine kosmetische Zusammensetzung ist.

15. Zusammensetzung nach dem vorhergehenden Anspruch, **dadurch gekennzeichnet, dass** sie ein transparentes Gel ist.

16. Kosmetisches Verfahren zum Abschminken und/oder zur Reinigung der Haut, der Haare, der Lippen und/oder der Augen, **dadurch gekennzeichnet, dass** auf die Haut, die Haare, die Lippen und/ oder die Augen eine Zusammensetzung nach einem der vorhergehenden Ansprüche aufgetragen wird.

17. Kosmetische Verwendung der Zusammensetzung nach einem der Ansprüche 1 bis 16 zum Abschminken und/oder zur Reinigung der Haut, der Haare, der Lippen und/oder der Augen.

18. Verwendung mindestens eines Proteinhydrolysats mit hydrophober Gruppe zur Stabilisierung eines Acylisethionats in einer wässrigen Zusammensetzung.

## Claims

1. Transparent aqueous composition comprising, in a physiologically acceptable medium, (i) at least one acylisethionate of formula (I):
RCO₂(CH₂)ₙSO₃⁻M⁺
in which R is a hydrocarbon-based radical containing from 6 to 26 carbon atoms, n is an integer from 2 to 4 and M is an alkali metal or alkaline-earth metal, the acylisethionate being present in an amount ranging from 0.5 to 10% by weight relative to the total weight of the composition,
and (ii) at least one protein hydrolysate containing a hydrophobic group, in an amount which is sufficient to dissolve the acylisethionate, the amount by weight of active material of protein hydrolysate containing a hydrophobic group being at least equal to the amount by weight of acylisethionate, and the protein hydrolysate being a protein hydrolysate of vegetable origin or a silk protein hydrolysate.

2. Composition according to the preceding claim, **characterized in that**, in formula (I), R is a cocoyl radical and M is sodium or potassium.

3. Composition according to either one of the preceding claims, **characterized in that** the hydrophobic group of the protein hydrolysate is an alkyl chain containing from 8 to 22 carbon atoms.

4. Composition according to any one of the preceding claims, **characterized in that** the hydrophobic group is a lauryl chain.

5. Composition according to any one of the preceding claims, **characterized in that** the protein hydrolysate containing a hydrophobic group is chosen from soybean, wheat and oat protein hydrolysates.

6. Composition according to the preceding claim, **characterized in that** the protein hydrolysate containing a hydrophobic group is chosen from, wheat protein hydrolysates modified with lauric acid and oat protein hydrolysates modified with lauric acid, the salts thereof and mixtures thereof.

7. Composition according to any one of the preceding claims, **characterized in that** the amount of protein hydrolysate containing a hydrophobic group ranges from 0.5 to 30% by weight of active material relative to the total weight of the composition.

8. Composition according to any one of the preceding claims, **characterized in that** water is present in an amount ranging from 40 to 95% by weight relative to the total weight of the composition.

9. Composition according to any one of the preceding claims, **characterized in that** it also contains another foam-forming surfactant.

10. Composition according to the preceding claim, **characterized in that** the foam-forming surfactant is a nonionic surfactant.

11. Composition according to the preceding claim, **characterized in that** the nonionic surfactant is chosen from the compounds of formula:
R¹O(G)ₚ
in which R¹ is a monovalent hydrocarbon-based chain containing from 1 to 30 carbon atoms, G is a sugar derivative containing 5 or 6 carbon atoms and p is an average statistical value ranging from 1 to 6.

12. Composition according to Claim 10 or 11, **characterized in that** the nonionic surfactant is chosen from decylglucoside and laurylglucoside, and mixtures thereof.

13. Transparent aqueous composition comprising, in a physiologically acceptable medium, (i) at least one acylisethionate of formula (I):
RCO₂(CH₂)ₙSO₃⁻M⁺
in which R is a hydrocarbon-based radical containing from 6 to 26 carbon atoms, n is an integer from 2 to 4 and M is an alkali metal or alkaline-earth metal, the acylisethionate being present in an amount ranging from 0.5 to 10% by weight relative to the total weight of the composition, (ii) at least one protein hydrolysate containing a hydrophobic group, in an amount which is sufficient to dissolve the acylisethionate, the amount by weight of active material of protein hydrolysate containing a hydrophobic group being at least equal to the amount by weight of acylisethionate, the protein hydrolysate being a protein hydrolysate of vegetable origin or a silk protein hydrolysate, and (iii) an alkylpolyglucoside.

14. Composition according to any one of the preceding claims, **characterized in that** it constitutes a cosmetic composition.

15. Composition according to any one of the preceding claims, **characterized in that** it constitutes a transparent gel.

16. Cosmetic process for removing make-up from and/or for cleansing the skin, the hair, the lips and/or the eyes, **characterized in that** a composition according to any one of the preceding claims is applied to the skin, the hair, the lips and/or the eyes.

17. Cosmetic use of the composition according to any one of Claims 1 to 15 for removing make-up from and/or for cleansing the skin, the hair, the lips and/or the eyes.

18. Use of at least one protein hydrolysate containing a hydrophobic group, the protein hydrolysate being a protein hydrolysate of vegetable origin, to stabilize an acylisethionate in an aqueous composition.
